(19) Europäisches Patentamt
European Patent Office
Office européen des brevets

(11) Veröffentlichungsnummer: **0 004 543**
**B1**

(12) # EUROPÄISCHE PATENTSCHRIFT

(45) Veröffentlichungstag der Patentschrift: **10.06.81**

(51) Int Cl.³: **C 07 C 27/00,** C 07 C 31/18, C 07 C 45/00, C 07 C 47/26, C 07 C 49/24, C 08 G 18/32, C 07 H 1/00

(21) Anmeldenummer: **79100415.3**

(22) Anmeldetag: **13.02.79**

(54) **Verfahren zur Herstellung von niedermolekularen Polyhydroxylverbindungen und ihre Verwendung zur Herstellung von Polyurethankunststoffen.**

(30) Priorität **25.02.78 DE 2808228**

(43) Veröffentlichungstag der Anmeldung: **17.10.79 Patentblatt 79/21**

(45) Bekanntmachung des Hinweises auf die Patenterteilung **10.06.81 Patentblatt 81/23**

(84) Benannte Vertragsstaaten **BE DE FR GB IT NL**

(56) Entgegenhaltungen
**DE-A-2 639 084**
**DE-A-2 714 084**
**DE-A-2 714 104**
**DE-A-2 721 186**

(73) Patentinhaber: **BAYER AG, Zentralbereich Patente, Marken und Lizenzen, D-5090 Leverkusen 1, Bayerwerk (DE)**

(72) Erfinder: **Stemmler, Ingo, Dr., Buschweg 21, D-5068 Odenthal (DE)**
Erfinder: **Müller, Hanns-Peter, Dr., Berta-von-Suttner-Strasse 40, D-5090 Leverkusen (DE)**
Erfinder: **Wagner, Kuno, Dr., Am Kiesberg 8, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 543 B1

BUNDESDRUCKEREI BERLIN

# Verfahren zur Herstellung von niedermolekularen Polyhydroxylverbindungen und ihre Verwendung zur Herstellung von Polyurethankunststoffen

Die vorliegende Erfindung betrifft ein verbessertes Verfahren zur Herstellung von Formose aus Formaldehyd. Die Verbesserung besteht darin, daß als Katalysator für die Kondensationsreaktion des Formaldehyds relativ geringe Mengen an basischen Bleiverbindungen eingesetzt werden, welche gleichzeitig zur Steuerung des pH-Werts dienen, so daß auf die Mitverwendung der bisher in diesem Zusammenhang üblichen organischen oder anorganischen Basen verzichtet werden kann.

Unter »Formose« werden erfindungsgemäß die an sich bekannten Gemische von niedermolekularen Polyhydroxyl-Verbindungen (mehrwertigen Alkoholen, Hydroxyaldehyden und Hydroxyketonen) verstanden, welche bei der Kondensation von Formaldehydhydrat entstehen.

Die Herstellung von Gemischen mehrwertiger Alkohole, Hydroxyaldehyde und Hydroxyketone aus Formaldehydhydrat wird in zahlreichen Literaturstellen beschrieben. Beispielsweise seien in diesem Zusammenhang Butlerow und Loew, Annalen 120, 295 (1861) bzw. J. pr. Chem. 33, 321 (1886); Pfeil, Chemische Berichte 84, 229 (1951); Pfeil und Schroth, Chemische Berichte 85, 303 (1952); R. D. Partridge und A. H. Weiss, Carbohydrate Research 24, 29—44 (1972); die Formosen aus Glycerinaldehyd bzw. Dioxyaceton nach Emil Fischer, die deutschen Patentschriften 822 385, 830 951 und 884 794, die US-Patentschriften 2 224 910, 2 269 935 und 2 272 378 sowie die englische Patentschrift 513 708 genannt.

Trotz all dieser Vorschläge zur Synthese von Polyhydroxylverbindungen durch Selbstkondensation von Formaldehyd ist aber bis zur Gegenwart kein technisch brauchbares Verfahren hierfür entwickelt worden, weil diese bekannten Verfahren des Standes der Technik mit gewissen Nachteilen behaftet sind, beispielsweise schlechte Raum-Zeit-Ausbeuten; gefärbte Nebenprodukte; mangelnde Reproduzierbarkeit der Hydroxylfunktionalität der Formosen; aufwendige Operationen zur Entfernung der als Hilfsreagentien eingesetzten Basen. Dies hat die Synthese von Polyhydroxylverbindungen durch Selbstkondensation des Formaldehydhydrats bisher unwirtschaftlich erscheinen lassen und verhindert, daß die Selbstkondensation des Formaldehydhydrats als Grundlage für ein technisches Verfahren — z. B. zur Synthese von mehrwertigen Alkoholen — herangezogen wurde.

Infolge der gleichzeitig ablaufenden Disproportionierung des Formaldehyds zu Methanol und Ameisensäure konnten mit den bisher bekannten Verfahren meist nur mäßige Ausbeuten erzielt werden, so daß die Aufarbeitung der entstehenden wäßrigen bzw. wäßrig/alkoholischen Formoselösungen erhebliche wirtschaftliche Kosten verursachte.

Bekanntlich wird die Disproportionierung von Formaldehyd in Methanol und Ameisensäure durch basische Verbindungen sehr stark katalysiert. Wie Pfeil, Chemische Berichte 84, 229 (1951), feststellte, hängt die Reaktionsgeschwindigkeit dieser sogenannten »Cannizzaro-Reaktion« vom Quadrat der Formaldehydkonzentration ab, während die Reaktionsgeschwindigkeit der Formaldehydpolyaddition (C—C-Verknüpfung) linear von der Formaldehydkonzentration abhängt (Pfeil und Schroth, Chemische Berichte 85, 303 [1952]). Mit steigender Aldehydkonzentration wird daher das Mengenverhältnis von gewünschten Polyhydroxylverbindungen zu Methanol und Ameisensäure zu Ungunsten der gesuchten Verbindungen verschoben. Daher wird in vielen zum Stand der Technik gehörenden Verfahren vorgeschlagen, die Kondensation des Formaldehyds zu Hydroxyaldehyden und Hydroxyketonen in Lösungen mit niedrigen Formaldehydkonzentrationen durchzuführen, um die Menge an Nebenprodukten so niedrig wie möglich zu halten. Zur Gewinnung der gebildeten Hydroxyaldehyde und Hydroxyketone ist es jedoch notwendig, das als Lösungsmittel verwendete Wasser wieder destillativ zu entfernen. Bedingt durch die hohe Verdampfungswärme des Wassers entstehen dadurch erhebliche Energiekosten. Verfahren zur Kondensation des Formaldehyds aus verdünnten wäßrigen Lösungen sind aus diesem Grunde unwirtschaftlich. Außerdem treten bei längeren Destillationszeiten in erheblichem Maß Zersetzungs- und Verfärbungsreaktionen der gebildeten Hydroxyaldehyde und Hydroxyketone auf.

Es ist daher wünschenswert, die Kondensation des Formaldehyds aus handelsüblichen konzentrierten Formalinlösungen durchzuführen, ohne daß dabei störende Nebenreaktionen auftreten.

Zur Vermeidung der Cannizzaro-Reaktion wurde weiter vorgeschlagen, Formaldehydlösungen in Gegenwart von Methanol, Äthanol oder anderen polaren organischen Lösungsmitteln zu kondensieren.

Durch Zugabe von organischen Lösungsmitteln wird jedoch der Formaldehydgehalt der Lösung wiederum verringert. Die zusätzlich erforderlichen Energiekosten zur Verdampfung des zugesetzten Lösungsmittels bei der Aufarbeitung der gebildeten Hydroxyaldehyde und -ketone lassen daher auch diese Verfahren unwirtschaftlich erscheinen. Zudem bilden sich aus Formaldehyd und niedrigen Alkoholen wenig stabile Halbacetale, die sich während der Kondensation unter spontaner Freisetzung der Alkohole zersetzen. Im Verlaufe von Kondensationsreaktionen, die bei Reaktionstemperaturen über dem Siedepunkt des betreffenden Alkohols durchgeführt werden, kommt es aus diesem Grund zu heftigen Siedeverzügen, insbesondere bei größeren Ansätzen, so daß die Kondensationsverfahren unter diesen Bedingungen technisch nicht gefahrlos durchgeführt werden können.

Der vorliegenden Erfindung liegt daher die Aufgabe zugrunde, ein technisch einfaches Verfahren bereitzustellen, nach welchem mit günstigen Raum-Zeit-Ausbeuten Gemische von Polyhydroxylverbindungen synthetisiert werden können, die weitgehend frei von Neben- und Zersetzungsprodukten sind. Die eingesetzten Hilfsreagentien (Katalysatoren, Basen) sollen sich auf einfache Weise vom Reaktionsprodukt abtrennen lassen. Die erhaltenen Gemische von Polyhydroxylverbindungen sollen farblos sein und keiner weiteren Reinigung bedürfen.

Aufgabe der vorliegenden Erfindung war es ferner, die Formaldehydselbstkondensation so zu steuern, daß sich die Produktverteilung der entstehenden Gemische von niedermolekularen Polyhydroxyverbindungen je nach Anwendungswunsch variieren und reproduzierbar einstellen läßt.

Die Lösung der gestellten Aufgabe bereitete jedoch aus den im folgenden dargelegten Gründen Schwierigkeiten. Die übliche bleikatalysierte Formosesynthese läuft nur ab, wenn der pH-Wert im Reaktionsgemisch mit zusätzlichen Basen auf bestimmte Werte eingestellt wird (vgl. GB-PS 513 708). Die hierfür bevorzugt eingesetzten Alkalihydroxide lassen sich aber aus dem Reaktionsprodukt nur mit großem Aufwand, beispielsweise mit Ionenaustauschern, entfernen. Auch die oft verwendeten tertiären Amine lassen sich im allgemeinen nur — selbst wenn sie wie Trimethylamin leicht flüchtig sind — mit Ionenaustauschern quantitativ von der Formose abtrennen (die während der Reaktion entstandenen Salze der Amine sind aus der Formose durch Destillation nicht zu entfernen). Die Verwendung von Ionenaustauschern zur Vollentsalzung der Formose ist jedoch wegen der hohen Abwassermengen unwirtschaftlich. Ein denkbarer Ausweg aus diesen Schwierigkeiten wäre die Verwendung basisch reagierender Metallverbindungen als Katalysator, da auf diese Weise die Menge der in die Formose eingebrachten Fremdionen niedrig bliebe.

Das bereits von O. LOEW (J. prakt. Chem. 33, 321 [1886]) für die Herstellung von Formose aus 4%igem wäßrigem Formaldehyd als Katalysatorbase verwendete Calciumhydroxid wäre sowohl aus ökologischen als auch aus ökonomischen Gründen hervorragend geeignet: $Ca(OH)_2$ katalysiert die Formosereaktion, reguliert gleichzeitig den pH-Wert des Reaktionsgemischs und ist — beispielsweise durch Fällungsreaktion mit Schwefelsäure — auf einfachem Wege vom Reaktionsprodukt als ungiftige Verbindung abtrennbar. Da aber $Ca(OH)_2$ nach E. PFEIL (Chem. Berichte 84, 229 [1951]) ein sehr wirksamer Katalysator für die Cannizzaro-Reaktion ist, müssen entweder Nebenreaktionen in Kauf genommen werden, was zu verringerten Ausbeuten bei der Formosebildung führt, oder man muß mit sehr verdünnten Formaldehydlösungen arbeiten, was aus wirtschaftlichen Gründen ebenfalls nachteilig ist.

Thalliumhydroxid soll nach E. PFEIL bzw. nach DE-PS 822 385 wesentlich bessere Ergebnisse bei der Formosesynthese liefern als $Ca(OH)_2$, da es die Formosebildung selektiv auf Kosten der Cannizzaro-Reaktion katalysiert. Die Ausbeuten dieses Verfahrens sind jedoch mit 70 bis 80% auch relativ gering. Darüber hinaus verbietet die extrem hohe Toxizität der Thalliumverbindungen deren technischen Einsatz.

Die dritte für die Formosesynthese bekannte Katalysatorbase ist $Pb(OH)_2$ bzw. PbO.

Gemäß DE-PS 564 678 wird aus einer 4%igen wäßrigen Formaldehydlösung unter Zugabe von 125 g $Pb(OH)_2$/kg HCHO ein Gemisch von $C_2$-, $C_3$- und $C_4$-Kohlenhydraten synthetisiert und anschließend zu den Polyalkoholen hydriert (65% Ausbeute). Die Aufarbeitung derartig verdünnter Reaktionsgemische erfordert jedoch neben einem hohen Energieverbrauch auch einen großen technischen Aufwand.

Neben 100 bis 150 g PbO/kg HCHO werden nach der US-Patentschrift 2 224 910 noch 1 bis 3 Gew.-% (bezogen auf HCHO) an zur Endiolbildung befähigten Verbindungen bei der Formosesynthese aus 10 bis 25%igen wäßrigen Formaldehydlösungen bei Reaktionsbeginn als Co-Katalysator zugesetzt (74—84% Ausbeute). Nach den Angaben dieser Literaturstelle liegt die effektivste Menge an Co-Katalysator zwischen 1 und 10 Gew.-% (bezogen auf wasserfreien Formaldehyd) und eine Vergrößerung des Anteils an Endiolbildnern auf über 10% bietet keinen Vorteil mehr.

Daher muß es als um so überraschender angesehen werden, daß, wie nunmehr gefunden wurde, der Einsatz von über 15 Gew.-% (bevorzugt über 20, besonders bevorzugt über 40 Gew.-%) an Co-Katalysator (Endiolbildner) für die Überführung konzentrierterer wäßriger Formaldehydlösungen (über 25 Gew.-%, bevorzugt 30 bis 70 Gew.-% HCHO) in Formose von besonderem Vorteil ist, da dann vorzugsweise nur noch ca. 20 bis 80 g, besonders bevorzugt 30 bis 60 g PbO/kg HCHO benötigt werden. Weiterhin wurde überraschenderweise gefunden, daß auch entsprechende molare Mengen anderer Blei-II-Verbindungen, welche noch weitaus schwächere basische Eigenschaften als das PbO besitzen, unter den Bedingungen des erfindungsgemäßen Verfahrens gute katalytische und genügend basische Eigenschaften aufweisen. Besonders überraschend ist jedoch, daß beim erfindungsgemäßen Verfahren (im Vergleich zum Stand der Technik) nur noch extrem geringe Basenäquivalente — in Form von basischen Blei-II-Verbindungen — eingesetzt zu werden brauchen.

Gegenstand der vorliegenden Erfindung ist somit ein Verfahren zur Herstellung von niedermolekularen Polyhydroxylverbindungen durch Selbstkondensation von Formaldehydhydrat in Gegenwart von löslichen oder schwer löslichen Verbindungen des zweiwertigen Bleis als Katalysator, deren gesättigte wäßrige Lösungen oder

Suspensionen einen pH-Wert von über 4,5, bevorzugt über 5,5, besonders bevorzugt über 6,0, aufweisen, welches dadurch gekennzeichnet ist, daß die Kondensationsreaktion in Gegenwart von

a) einer Menge an Katalysator, welche 0,1 bis 1, vorzugsweise 0,2 bis 0,8, besonders bevorzugt 0,3 bis 0,6, Grammäquivalenten Blei pro kg Formaldehyd entspricht,
b) mehr als 15 Gew.-%, bevorzugt über 20 Gew.-%, besonders bevorzugt über 40 Gew.-% (bezogen auf Formaldehyd) an zur Endiolbildung befähigten Verbindungen als Co-Katalysator und gegebenenfalls
c) von beliebigen niedermolekularen und/oder höhermolekularen Polyhydroxylverbindungen

durchgeführt wird, und daß in Abwesenheit weiterer organischer oder anorganischer Basen gearbeitet wird.

Wegen der weitgehenden Zurückdrängung der gekreuzten Cannizzaro-Reaktion werden erfindungsgemäß in bis zu 95—98%iger Ausbeute mit hoher Reproduzierbarkeit der durchschnittlichen OH-Funktionalität hochkonzentrierte wäßrige Lösungen von Polyolen, Hydroxyaldehyden und Hydroxyketonen erhalten, die völlig farblos sind und daher keiner weiteren Reinigung und Entfärbung bedürfen, während, wie schon erwähnt, bei den Verfahren des Standes der Technik aufgrund von Zersetzungsreaktionen häufig stark gefärbte, störende Nebenprodukte gebildet werden, deren Entfernung nicht oder nur mühsam mit großem zusätzlichem Aufwand gelingt.

Die Selbstkondensation des Formaldehydhydrats unter Bildung von Hydroxyaldehyden und Hydroxyketonen wird erfindungsgemäß durch schwerlösliche, ebenso wie durch in Wasser lösliche Verbindungen (insbesondere Salze) des zweiwertigen Bleis katalysiert.

Erfindungswesentlich ist in diesem Zusammenhang lediglich, daß mit vollentsalztem, $CO_2$-freiem Wasser hergestellte gesättigte Lösungen bzw. Suspensionen dieser Blei-II-Verbindungen einen pH-Wert von $\geq 4,5$, bevorzugt $\geq 5,5$ aufweisen. Im allgemeinen werden erfindungsgemäß ca. 1 bis 10 Gew.-%, bevorzugt 2 bis 8 Gew.-%, bezogen auf eingesetzten Formaldehyd, an Blei-II-Ionen angewandt.

Erfindungsgemäß besonders bevorzugte »Katalysatorbasen« sind Bleioxid (bevorzugt die gelbe Modifikation), Bleihydroxid, Bleiweiß (basisches Bleicarbonat), Bleicarbonat, Bleiacetat, basisches Bleiacetat, alle basischen Bleisalze (z. B. PbX(OH), wobei X für ein einwertiges Anion steht) sowie Gemische von zwei oder mehr der vorgenannten Katalysatorbasen. Daneben können aber auch andere basische Blei-II-Verbindungen, z. B. Blei-phenolate und Bleithiophenolate verwendet werden.

An Stelle des leicht verfügbaren, billigen PbO können unter den gleichen oder nur wenig abgewandelten experimentellen Bedingungen auch die ebenfalls schwerlöslichen Verbindungen $Pb(OH)_2$ und $PbCO_3$ oder basische Bleicarbonate (z. B. Bleiweiß, $Pb(OH)_2 \cdot 2\,PbCO_3$) als Katalysatorbasen eingesetzt werden. Diese schwerlöslichen Bleibasen können direkt als Pulver, in Form von rührbaren Suspensionen (z. B. in Wasser, Formalin, Formose oder hydrierter Formose, Alkoholen sowie Gemischen dieser Verbindungen) oder als Pasten (aufgeschlämmt in den vorgenannten Flüssigkeiten) zum Reaktionsgemisch zugesetzt werden. Dagegen werden leichtlösliche Blei-II-Katalysatorbasen entweder als Pulver oder bevorzugt in Form von Lösungen (in Wasser, Formalin, Alkoholen o. ä.) dem Reaktionsmedium zugemischt. Neben dem sich hierfür vorzugsweise anbietenden Blei-II-acetat sind Lösungen basischer Blei-II-salze oder Lösungen von PbO bzw. $Pb(OH)_2$ in bleisalzhaltigen Lösungen besonders bevorzugt (Blei-II-Salzlösungen sind vorteilhafterweise in der Lage, beträchtliche Anteile des sonst schwerlöslichen Blei-II-oxids bzw. -hydroxids aufzulösen). Oberhalb eines gewissen PbO- bzw. $Pb(OH)_2$-Gehaltes reagieren diese Lösungen ausreichend alkalisch und stellen daher eine vorzügliche, leicht dosierbare Anwendungsform der Blei-II-Katalysatorbasen dar. Ganz besonders bevorzugt sind in diesem Zusammenhang Lösungen von PbO bzw. $Pb(OH)_2$ in wäßrigen Blei-II-acetatlösungen (sogenannter »Bleiessig«, Gmelins Handbuch der Anorganischen Chemie, 8. Auflage, System Nr. 47, Verlag Chemie, Weinheim/Bergstr. 1969; S. 772 und 775).

Die Lösungen der genannten basischen Blei-II-salze sowie Lösungen von PbO bzw. $Pb(OH)_2$ in Blei-II-salz-Lösungen eignen sich wegen ihrer leichten Dosierbarkeit zur pH-Steuerung der kontinuierlichen, aber auch der diskontinuierlichen Formosesynthese besonders gut. Selbstverständlich kann aber erfindungsgemäß die pH-Steuerung während der Selbstkondensation des Formaldehydhydrats auch mit den anderen weiter obengenannten schwerlöslichen und löslichen Pb-II-Verbindungen in den beschriebenen Anwendungsformen erfolgen.

Man läßt erfindungsgemäß die Selbstkondensation des Formaldehydhydrats in Gegenwart von zur Endiolbildung befähigten Verbindungen als Co-Katalysator ablaufen. Es werden dabei über 15, vorzugsweise über 20, besonders bevorzugt über 40 Gew.-%, bezogen auf Formaldehyd, an zur Endiolbildung befähigten Verbindungen eingesetzt.

Prinzipiell kommen für diesen Zweck beliebige an sich bekannte Verbindungen in Frage, welche in $\alpha$-Stellung zu einer Carbonylgruppe eine Hydroxylgruppe enthalten, wie sie z. B. auch in der eingangs zitierten Literatur als Co-Katalysatoren beschrieben sind; erfindungsgemäß bevorzugte Co-Katalysatoren sind jedoch Formose selbst und Oxidationsprodukte von mehrwertigen Alkoholen mit Hydroxylgruppen an einander benachbarten C-Atomen (der zuletzt genannte Typ von Co-Katalysator wird in der DE-OS

2 714 084 eingehend beschrieben).

Neben den zur Endiolbildung befähigten Verbindungen können im erfindungsgemäßen Verfahren auch Polyhydroxylverbindungen in einer Menge bis zu 200 Gew.-%, vorzugsweise 10 bis 100 Gew.-%, bezogen auf eingesetzten Formaldehyd, mitverwendet werden. Verbindungen dieser Art werden z. B. in der DE-OS 2 714 104 beschrieben.

Die erfindungsgemäß mitzuverwendende Menge an Co-Katalysator ist (bezogen auf im Reaktionsgemisch vorhandenen Formaldehyd) nach oben hin im Prinzip nicht begrenzt und nimmt, wie leicht einzusehen ist, im Verlaufe der Kondensationsreaktion (wegen der Neubildung von Formose und des Verbrauchs an Formaldehyd) zu; sie kann insbesondere bei der unten beschriebenen kontinuierlichen Arbeitsweise, bei welcher der Formaldehyd dem Reaktionsgemisch kontinuierlich oder portionsweise zugegeben wird, schon anfangs sehr hoch sein. Aus praktischen Gründen wird jedoch die Co-Katalysatormenge am Beginn der Kondensationsreaktion die 40fache Menge an vorhandenem Formaldehyd im allgemeinen nicht überschreiten.

Wie schon erwähnt, ist die Verfahrensführung bei der erfindungsgemäßen Kondensation von Formaldehydrat in Gegenwart von basischen Blei-II-Verbindungen weitgehend unkritisch.

Es wird bei Temperaturen über 70° C, vorzugsweise bei 85—150° C, besonders bevorzugt bei 91—120° C, und in einem pH-Bereich von 3 bis 8, bevorzugt 3 bis 7,5, besonders bevorzugt 3,2 bis 7, gearbeitet. Im allgemeinen setzt man erfindungsgemäß 25 bis 75 Gew.-%, vorzugsweise 30 bis 70 Gew.-%, besonders bevorzugt 35 bis 65 Gew.-%, Formaldehyd enthaltende wäßrige und/oder alkoholische Formaldehydlösungen und/oder Paraformaldehyddispersionen ein. Ebensogut lassen sich aber auch formaldehydhaltige Synthesegase in cokatalysatorhaltige Absorptionsflüssigkeiten einleiten und in diesen Lösungen in Gegenwart der vorgenannten erfindungsgemäßen Blei-II-Katalysatorbasen in situ oder auch nach Abschluß des Absorptionsvorganges zu Formose kondensieren. Je nach dem Säuregehalt dieser Ausgangskomponenten richtet sich die erfindungsgemäß zuzusetzende Menge der Katalysatorbase innerhalb der oben angegebenen Bereiche.

Man kann erfindungsgemäß die Gesamtmenge der Bleiverbindung gleich am Beginn der Kondensationsreaktion zufügen, da infolge der weitgehenden Unterdrückung von (gekreuzten) Cannizzaro-Reaktionen während der Formosebildung nur wenig Säuren entstehen, so daß der pH-Wert während der Kondensationsreaktion nur langsam abnimmt.

In manchen Fällen (vor allem beim unten beschriebenen kontinuierlichen Verfahren) ist es zweckmäßig, die Katalysatorbase portionsweise oder kontinuierlich zuzufügen. Durch die Menge und die Art der Zugabe der Bleiverbindungen hat man es auch in der Hand, die Reaktionsgeschwindigkeit in der gewünschten Weise zu beeinflussen.

Das erfindungsgemäße Verfahren der Formosesynthese aus Formaldehydhydrat kann sowohl kontinuierlich als auch diskontinuierlich ausgestaltet werden.

Eine bevorzugte Ausführungsform des Verfahrens geht von methanolfreiem, handelsüblichem ca. 37%igem Formalin aus, welches als Stabilisator beispielsweise Polyvinylalkohol enthält. Das Formalin wird unter Rühren auf 95—98° C erwärmt, mit der erfindungsgemäß erforderlichen Menge einer im allgemeinen ca. 30- bis 70gew.-%igen Lösung des Co-Katalysators in Formalin oder Wasser versetzt und erneut auf 95 bis 98° C erhitzt. Die Heizquelle wird entfernt und anschließend wird im Verlauf von 1 bis 5 Minuten die gesamte vorgesehene Menge an gelbem Blei-II-oxid in Pulverform (bzw. einer anderen basischen Pb-Verbindung) direkt ins Reaktionsgemisch eingetragen, so daß keine PbO-Reste an der Gefäßwand haftenbleiben. (Die PbO-Reste außerhalb der flüssigen Phase werden nämlich durch Formaldehyddämpfe teilweise oder völlig zu metallischem Blei reduziert, welches ins Reaktionsmedium gespült werden kann. Wegen der mangelhaften Löslichkeit der Pb-Teilchen ist dann die Formose im allgemeinen durch schwarze Teilchen getrübt und verfärbt. Darüber hinaus kondensiert Formaldehyd aus der Gasphase an dem feuchten PbO außerhalb der flüssigen Phase zu braunen Produkten, welche die Formose verfärben und nur schwierig zu entfernen sind). Die Formosereaktion springt sofort an, und das Reaktionsgemisch erwärmt sich zum Sieden (meist 99 bis 109° C). Die Umsetzung verläuft bis zum Reaktionsende exotherm. Nach insgesamt etwa 5 bis 30 Minuten klärt sich das anfangs weiße, milchig trübe Reaktionsgemisch, da das PbO, welches ursprünglich zu einem großen Teil suspendiert vorlag, mit der Zeit in Lösung geht. Nach insgesamt 10 bis 150 Minuten, bevorzugt 15 bis 110 Minuten, besonders bevorzugt 20 bis 75 Minuten, wird die Reaktion bei einem Restformaldehydgehalt von 0 bis 8 Gew.-%, bevorzugt 0,2 bis 4 Gew.-%, besonders bevorzugt 0,4 bis 2 Gew.-% durch Kühlen und/oder Zusatz von Säure, gegebenenfalls unter gleichzeitigem Entfernen der Katalysatorbase abgebrochen. Bei dieser diskontinuierlichen Verfahrensweise stellt sich der pH des Reaktionsgemisches von selbst ein und es kann auf eine externe pH-Steuerung verzichtet werden. (Im allgemeinen liegt der pH-Wert des Reaktionsgemisches anfangs zwischen 4,0 und 7,5, bevorzugt zwischen 4,5 und 6,5, nach 10 bis 30 Prozent Umsatz zwischen 4,0 und 6,5, bevorzugt zwischen 4 und 6 und nach 60 bis 95 Prozent Umsatz zwischen 3 und 5,5, bevorzugt 3,5 bis 5).

Selbstverständlich können auch methanolhaltige Formaldehydlösungen zur erfindungsgemäßen Formoseherstellung verwendet werden. Die Reaktionstemperaturen liegen dabei — je nach Methanolgehalt des Formalins und dadurch

bedingte Siedepunktserniedrigung — entsprechend tiefer. Auch paraformaldehydhaltige bzw. 1,3,5-trioxanhaltige wäßrige Lösungen oder Suspensionen können erfindungsgemäß als Ausgangsmaterial für die Formosesynthese dienen.

Eine weitere bevorzugte Variante des erfindungsgemäßen Verfahrens mit im allgemeinen besonders niedrigem Verbrauch an Blei-II-Verbindungen — bezogen auf eingesetzten HCHO — besteht in der halbkontinuierlichen bzw. kontinuierlichen Durchführung der Formosesynthese unter Einsatz hoher Mengen an Co-Katalysator während der Startphase der Reaktion. Zunächst wird die Reaktion unter ähnlichen bzw. identischen Bedingungen wie beim diskontinuierlichen Verfahren gestartet und bis zu dem gewünschten Umsetzungsgrad bzw. Restformaldehydgehalt geführt. Vorzugsweise wird der pH hierbei aber durch portionsweise oder kontinuierliche Zugabe der Katalysatorbase auf einem bestimmten Wert oder innerhalb eines bestimmten pH-Bereiches (zwischen 3,5 und 6,5, vorzugsweise zwischen 3,8 und 5,8) gehalten. Nach Ablauf dieser diskontinuierlichen Startphase wird dem Reaktionsgemisch kontinuierlich oder portionsweise Formaldehyd zugeführt und gleichzeitig die äquivalente Menge an Reaktionsprodukt chargenweise oder kontinuierlich entnommen. Daneben wird die Katalysatorbase in einer solchen Weise zudosiert, daß der gewünschte pH-Wert erhalten bleibt. Je nach der Temperatur und der Menge pro Zeiteinheit der zudosierten Ausgangskomponenten kann es gegebenenfalls erforderlich sein, das Reaktionsgemisch zu kühlen oder zu heizen. Ein besonderer Vorteil des erfindungsgemäßen Verfahrens liegt darin, daß man, abgesehen von der Reaktionstemperatur, nur zwei Parameter beachten muß, nämlich die Zufuhr an Formaldehyd und an Katalysatorbase. Durch Variation dieser beiden Größen werden der pH-Wert und die Verweilzeit des Reaktionsgemisches in der Reaktionszone und damit auch der Restformaldehydgehalt bestimmt. Die Produktverteilung in der so erhaltenen Formose ist auf diese Weise leicht in weiten Grenzen variierbar und reproduzierbar. Der pH-Wert wird während der Reaktion im allgemeinen zwischen 3,5 und 6,5, bevorzugt zwischen 3,8 und 5,8 und besonders bevorzugt zwischen 3,8 und 5 gehalten. Der Durchsatz an Formaldehydhydrat beträgt — bezogen auf ein Reaktorvolumen von 1 l — 0,05 bis 5, bevorzugt 0,1 bis 2, besonders bevorzugt 0,2 bis 1 kg/Stunde. Die Temperatur in der Reaktionszone liegt im allgemeinen über 70°C, bevorzugt zwischen 85°C und 150°C, besonders bevorzugt zwischen 91 und 120°C, und damit oft bei der Siedetemperatur des Reaktionsgemisches, in vielen Fällen aber auch darunter. Nach Verlassen der Reaktionszone wird das Produkt gekühlt und/oder mit Säure versetzt, um die Reaktion abzubrechen, wobei gleichzeitig gegebenenfalls der Bleikatalysator ausgefällt wird. Als Apparaturen für diese (halb) kontinuierliche Verfahrensvariante kommen beispielsweise Rührkesselkaskaden in Frage, welche kontinuierlich oder auch halbkontinuierlich betrieben werden können. Auf ähnlich günstige Weise gelingt die Herstellung von Formose nach dem erfindungsgemäßen Verfahren auch in einem kontinuierlich betriebenen Reaktionsrohr, in welches zur Aufrechterhaltung des gewünschten pH-Wertes im gesamten Reaktionsvolumen an einer oder mehreren Stellen des Rohres kontinuierlich die Katalysatorbase in der notwendigen Menge hinzugefügt werden kann. Auch in diesem Fall ist es möglich, durch Variation der Durchflußzeiten und der pH-Führung die Produktverteilung der Formose in weiten Grenzen zu verändern.

Eine besonders bevorzugte und wirtschaftliche Ausführungsform des erfindungsgemäßen Verfahrens besteht darin, Formose direkt, d. h. ohne den Umweg über wäßrige Formalinlösungen oder Paraformaldehyd, aus Formaldehyd enthaltenden Synthesegasen herzustellen. Man leitet zu diesem Zweck die Synthesegase, wie sie bei der großtechnischen Herstellung von Formaldehyd anfallen, (vorzugsweise ohne jede vorgeschaltete Reinigung), vorzugsweise bei Temperaturen zwischen 70 und 150°C kontinuierlich oder diskontinuierlich in eine Absorptionsflüssigkeit, welche aus Wasser, gegebenenfalls ein- oder mehrwertigen niedermolekularen Alkoholen und/oder höhermolekularen Polyhydroxylverbindungen, den zur Endiolbildung befähigten Verbindungen als Co-Katalysator und der löslichen oder unlöslichen Blei-II-Verbindungen als Katalysatorbase besteht und einen pH-Wert von 3 bis 8, vorzugsweise 3,5 bis 6,5, aufweist, kondensiert den Formaldehyd direkt in situ in der Absorptionsflüssigkeit (gegebenenfalls auch in einem nachgeschalteten Reaktionsrohr bzw. einer nachgeschalteten Rührkesselkaskade), bricht die Selbstkondensation des Formaldehyds bei einem Restformaldehydgehalt im Reaktionsgemisch von 0 bis 10 Gew.-% durch Kühlen und/oder durch Desaktivierung des Katalysators mittels Säuren ab und entfernt schließlich den Katalysator.

Beim erfindungsgemäßen Verfahren werden ausschließlich bleihaltige Katalysatorbasen eingesetzt. Aus ökologischen Gründen sollten Blei-II-Ionen aber bereits aus Rohprodukten abgetrennt werden, bevor diese zum Verkauf gelangen. Diese Forderung läßt sich einfach und ohne großen technischen oder finanziellen Aufwand erfüllen.

Eine bevorzugte Methode besteht in der elektrolytischen Abscheidung der Blei-II-Ionen als elementares Blei. Hierbei sind beispielsweise mit Suspensionselektroden Restwerte an Schwermetallionen von 1 ppm oder darunter erreichbar (vgl. das Verfahren zur Entfernung gelöster Schwermetallverbindungen aus Abwässern, wie es in European Chemical News Vol. 31, No. 805, S. 24, [1977] beschrieben wird).

Das abgeschiedene Blei kann nach Umwandlung in eine Blei-II-Verbindung wieder als Katalysatorbase eingesetzt werden. Durch Behandlung mit Luftsauerstoff bei erhöhter Temperatur wird Blei, meist in Form seiner Schmelze (Schmelzpunkt 328°), großtechnisch in PbO überführt. Bleibt man mit der Temperatur unterhalb des Schmelzpunktes von Bleioxid (ca. 884°C), so fällt PbO als lockeres, gelbes Pulver an (H. Remy, Lehrbuch d. Anorganischen Chemie, 12. Auflage, Leipzig 1965, Band I, S. 665), welches besonders gute katalytische Eigenschaften für das erfindungsgemäße Verfahren aufweist. Das Recycling des metallischen Bleis kann aber auch durch Umwandlung auf nassem Wege erfolgen; z. B. löst sich Blei in lufthaltiger Essigsäure leicht auf und kann hieraus nach Einengen oder Kristallisation bei tiefer Temperatur als katalytisch aktives Bleiacetat erneut dem Formoseprozeß zugeführt werden. Da die Mutterlauge des Bleiacetats, gegebenenfalls nach Zusatz weiterer Essigsäure, immer wieder zum Auflösen von Blei benutzt werden kann, fallen bei der beschriebenen Kreisführung des Bleikatalysators keinerlei bleihaltige Abfallprodukte an.

Eine weitere Möglichkeit besteht in der elektrolytischen Abscheidung von Blei-II-Ionen als $PbO_2$ oder gleichzeitig als Pb und als $PbO_2$. Da Bleidioxid bereits bei gelindem Erwärmen Sauerstoff abspaltet, kann es leicht wieder auf trocknem Wege in katalytisch aktives PbO überführt werden.

Eine andere bevorzugte Arbeitsweise bei der Entfernung der Katalysatorbase aus der Rohformose ist die Ausfällung der Blei-II-Ionen als schwerlösliches Salz. Im Prinzip könnten als Fällreagentien auch Salze, z. B. Soda, Ammoniumcarbonat, Natriumhydrogencarbonat u. a., eingesetzt werden. Da hierbei aber die Kationen als neue Verunreinigung in der Formose verbleiben, ist ein Zusatz von Salzen nachteilig. Vorzugsweise werden daher äquivalente Mengen oder ein geringer Überschuß an Säuren zur Fällung des Bleis eingesetzt, wobei vorteilhafterweise gleichzeitig der pH-Wert im Rohprodukt absinkt, so daß eine weitere Kondensation der Carbonylverbindungen im Produkt unterbunden wird. Prinzipiell können alle Säuren, welche mit Blei-II-Ionen schwerlösliche Salze bilden, wie z. B. Oxalsäure, Schwefelsäure, Phosphorsäure oder Schwefelwasserstoff, eingesetzt werden.

Besonders bevorzugt ist der Einsatz von Oxalsäure, da Bleioxalat sich bereits oberhalb 320°C zu einem Gemisch von Pb und PbO zersetzt. Aus Formoselösungen mit hohem Alkoholgehalt (z. B. Methanol oder Äthanol) können Blei-II-Ionen — falls der Wasseranteil in der Lösung klein genug ist — auch als Formiat bei Raumtemperatur gefällt werden. Auch das Bleiformiat ist thermisch leicht zu Pb und PbO zu zersetzen.

Auf diese Weise ist eine vollkommene Ausnützung des zur Katalyse benötigten Bleis möglich, ohne daß fortwährend neue Mengen an Blei gebraucht und schädliche Abfallprodukte gebildet werden. Diese Verfahrensvariante ist daher aus ökonomischen sowie ökologischen Gründen von besonderem Interesse.

Nach diesem Reinigungsschritt sind in der erfindungsgemäß hergestellten Formose vorteilhafterweise nur noch unbedeutende Spuren an kationischen Verunreinigungen enthalten. Es ist also nicht notwendig, Kationenaustauscherharze zur Entfernung von Ionen, welche auf anderem Wege nicht abgetrennt werden können (z. B. Alkali oder $NH_4^+$) einzusetzen. Damit wird auch das häufige Regenerieren großer Austauscherharzmengen, welches stets mit großen Abwassermengen verbunden ist, vermieden.

Eventuell im Reaktionsprodukt noch nach der Fällung (oder Elektrolyse) verbliebene Pb-Spuren (im ppm-Maßstab) können hingegen ohne Aufwand mit kleinen Mengen an Kationenaustauschern entfernt werden. Ein solcher zusätzlicher Reinigungsschritt erübrigt sich jedoch in den meisten Fällen.

Während der Formosereaktion entstehen auch geringe Mengen an organischen Säuren (z. B. Ameisensäure, Milchsäure und Zuckersäure). Bei vielen Anwendungszwecken stören diese nicht; manchmal ist es jedoch zweckmäßig, alle Anionen aus der Formose zu entfernen. Leichtflüchtige Säuren, beispielsweise Ameisensäure, können direkt oder nach saurer Veresterung abdestilliert werden. Ameisensäure kann darüber hinaus auch katalytisch zersetzt werden. Die restlichen Anionen lassen sich mittels Anionenaustauschern entfernen.

Die erfindungsgemäß hergestellten Formosen können nachträglich auch durch überschüssigen Formaldehyd in ihre Halbacetale übergeführt oder durch Reaktion mit Formaldehyd in Gegenwart von Basen $\alpha$-methyloliert werden.

In Abhängigkeit von den Reaktionsbedingungen und von der Reaktionsführung bei der Formaldehydkondensation lassen sich die Eigenschaften der Formose in weiten Grenzen variieren. Im allgemeinen ist das mittlere Molekulargewicht und damit die Hydroxylfunktionalität der Formosen um so höher, je weiter die Kondensationsreaktion geführt wird, d. h. je weniger Restformaldehyd beim Abbruch der Kondensationsreaktion noch vorliegt. Bei einem Restformaldehydgehalt von 0 bis 1,5 Gew.-% wird eine Formose erhalten, welche ca 25—35 Gew.-% an Anteilen mit 5 C-Atomen, 30—45 Gew.-% an Verbindungen mit 6 C-Atomen und ca. 10—20 Gew.-% an Verbindungen mit 7 und mehr C-Atomen enthält. Dagegen werden zusammen nur ca. 15—20% an Polyolen, Hydroxyketonen und Hydroxyaldehyden mit 2,3 und 4 C-Atomen erhalten. Dies entspricht einer mittleren Hydroxylfunktionalität von ca. 5.

Durch Abbruch der Formaldehydselbstkondensation bei etwas höheren Restformaldehydgehalten werden andere Komponentenverteilungen erhalten. So ergibt sich bei einem Abbruch der Kondensationsreaktion bei 2 bis 2,5% Formaldehydgehalt ein Gemisch mehrwertiger Alkohole, Hydroxyaldehyde und Hydroxyketone mit einer mittleren Hydroxylfunktionalität von ca. 4. Noch andere Komponentenverteilungen mit weiter erniedrigter durchschnittlicher Hydroxylfunktionalität werden erhalten, wenn man die Kondensationsreaktion bei Restformaldehydgehalten abbricht, die noch höher liegen als 2,5.

Zusammenfassend läßt sich feststellen, daß das erfindungsgemäße Verfahren gegenüber den Verfahren des Standes der Technik folgende wesentliche Vorteile bietet:

1. Das erfindungsgemäße Verfahren liefert Gemische von Hydroxyaldehyden, Hydroxyketonen und mehrwertigen Alkoholen ohne störende Zersetzungsprodukte.
2. Das erfindungsgemäße Verfahren liefert Formosen mit unterschiedlicher OH-Funktionalität, deren Verteilung je nach Anwendungszweck gezielt variierbar ist. Insbesondere können Gemische hergestellt werden, die über 90 Gew.-%, besonders bevorzugt über 95 Gew.-%, an Verbindungen mit mehr als 4 Kohlenstoffatomen enthalten. Auch die hohe Reproduzierbarkeit der Produktverteilung stellt einen wesentlichen Vorteil gegenüber den Verfahren des Standes der Technik dar.
3. Beim erfindungsgemäßen Verfahren werden farblose Produkte erhalten, die — nach Entfernung der Katalysatorbase — ohne weitere Reinigung direkt hydriert werden können oder für die anderen unten geschilderten Anwendungszwecke einsetzbar sind.
4. Das erfindungsgemäße Verfahren ist gegenüber den Verfahren des Standes der Technik besonders wirtschaftlich. Infolge der möglichen Verwendung von hochkonzentrierten Formaldehydlösungen oder formaldehydhaltigen Synthesegasen werden zusätzliche Energiekosten für die Verdampfung des Lösungsmittels vermieden. Da beim erfindungsgemäßen Verfahren praktisch keine störenden unerwünschten Nebenreaktionen auftreten, werden Ausbeuten von 95—98%, bezogen auf eingesetzten Formaldehyd erreicht.
   Das erfindungsgemäße Verfahren verläuft außerdem im Vergleich zu den bekannten Verfahren des Standes der Technik äußerst rasch und ermöglicht daher extrem hohe Raum-Zeit-Ausbeuten.
5. Die beim erfindungsgemäßen Verfahren eingesetzten bleihaltigen Katalysatorbasen können nach ihrer Verwendung direkt oder nach einem einfachen Aufarbeitungsschritt wieder verwendet werden, so daß keine ökologisch bedenklichen bleihaltigen Abfälle anfallen.

Die erfindungsgemäß erhaltenen Formosen bzw. die daraus durch Hydrierung (siehe z. B. DE-OS 2 714 084 und DE-OS 2 714 104) erhaltenen Polyolgemische (»Formite«) sind wertvolle Ausgangsmaterialien für eine Vielzahl anwendungstechnisch interessanter Produkte; insbesondere sind sie als Polyolkomponente bei der Herstellung von Polyurethankunststoffen geeignet.

Gegenstand der Erfindung ist somit auch ein Verfahren zur Herstellung von gegebenenfalls zellförmigen Polyurethankunststoffen durch Umsetzung von

A) Polyisocyanaten mit
B) niedermolekularen Polyhydroxylverbindungen sowie gegebenenfalls
C) höhermolekularen Polyhydroxylverbindungen, weiteren Kettenverlängerungsmitteln, Treibmitteln, Katalysatoren und weiteren an sich bekannten Zusatzstoffen, welches dadurch gekennzeichnet ist, daß als Komponente B die erfindungsgemäß erhaltenen Formosen und/oder deren Hydrierungsprodukte eingesetzt werden.

Für die Herstellung der Polyurethan-Kunststoffe geeignete Polyisocyanate, höhermolekulare Polyhydroxylverbindungen, Kettenverlängerungsmittel, Treibmittel, Katalysatoren und weitere Zusatzstoffe werden z. B. in den Deutschen Offenlegungsschriften 2 714 084 und 2 714 104 beschrieben, auf welche in diesem Zusammenhang verwiesen sei.

Die ausschließliche Umsetzung der erfindungsgemäß zugänglichen Polyhydroxylverbindungen (ohne Mitverwendung anderer gegenüber Isocyanaten reaktiver Komponenten) mit stark elastifizierenden Polyisocyanaten, wie z. B. Polyisocyanaten mit Biuretstruktur (DE-AS 1 543 178) führt zu harten, lichtechten, kratz- und lösungsmittelfesten Beschichtungen und Lakken.

Durch Propoxylierung oder/und Oxäthylierung der Formose bzw. der Formite lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden.

Durch Umsetzung der erfindungsgemäß hergestellten Formose bzw. des daraus durch Hydrierung erhaltenen Formits mit mehrwertigen Carbonsäuren der obengenannten Art, z. B. Phthalsäure, Isophthalsäure, Terephthalsäure, Tetra- und Hexahydrophthalsäure, Adipinsäure oder Maleinsäure, nach den üblichen Verfahren der Polyesterkondensation, wie sie beispielsweise in Houben-Weyl, Methoden der organischen Chemie, Bd. XIV 12, S. 40 beschrieben sind, lassen sich stark verzweigte Polyester synthetisieren, die als Zusätze zu Alkydharzen deren Härte verbessern. Die Hydroxylgruppen enthaltenden Polyester, die aus den erfindungsgemäß hergestellten Hydroxylverbindungen synthetisiert werden, sind selbstverständlich ebenfalls als Ausgangskomponente zur Herstellung von Polyurethankunststoffen brauchbar.

Die erfindungsgemäß hergestellten Formosen und Formite lassen sich auch sehr leicht mit langkettigen, aliphatischen Monocarbonsäuren, wie Capryl-, Caprin-, Laurin-, Myristin-, Palmitin-,

Stearin-, Öl-, Linol-, Arachidon-, oder Behensäure, sowie deren Derivaten, wie z. B. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte der Polyole oder auch Umsetzungsprodukte der erfindungsgemäß zugänglichen Polyhydroxylverbindungen mit langkettigen Monoisocyanaten, wie n-Octyl-, n-Decyl-, n-Dodecyl-, Myristyl-, Cetyl- oder Stearylisocyanat zu Carbamidsäureestern (siehe z. B. K. Lindner, Tenside Bd. III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können.

Die erfindungsgemäßen Formosen und Formite lassen sich auch als Feuchthaltemittel in Kosmetika und Kunststoffen verwenden. Sie können aber z. B. auch als Gefrierschutzmittel dienen.

Ebenso ist ihr Einsatz als kohlenhydrathaltiges Substrat in Nährböden von Mikroorganismen möglich. Hierzu haben sich besonders diejenigen Verfahrensprodukte bewährt, die hauptsächlich aus 5 und 6 Kohlenstoffatome enthaltenden Hydroxyaldehyden und Hydroxyketonen bestehen.

Die folgenden Beispiele erläutern das erfindungsgemäße Verfahren. Wenn nicht anders vermerkt, sind Zahlenwerte als Gewichtsteile bzw. Gewichtsprozente zu verstehen.

### Beispiel 1

3000 g einer 37%igen wäßrigen Formaldehydlösung werden unter Rühren auf 93° C erhitzt und mit 200 g einer 75%igen wäßrigen, entsalzten Formoselösung (hergestellt nach DE-PS 884 794) vermischt. Bei 96° C Innentemperatur werden 30 g PbO auf einmal zugegeben.

Die Temperatur des Gemisches steigt auf 99° C, und die Formosereaktion springt an. Nach 10 Minuten werden weitere 25 g PbO ins Reaktionsgemisch eingetragen. Bei einem Restformaldehydgehalt von 1,5% wird die Reaktion nach insgesamt 55 Minuten durch Kühlen mit Eiswasser abgebrochen. Bei Raumtemperatur wird das Reaktionsgemisch mit 180 g 20%iger Sodalösung versetzt, 10 Minuten gerührt und anschließend vom Bleicarbonat abgesaugt. Nach Vollentsalzung über Ionenaustauscher erhält man in 92% Ausbeute eine Formose mit 71,2% Zucker (berechnet als Glukose) bei 4,8% Wassergehalt. Komponentenverteilung nach katalytischer Hydrierung (in %):

| $C_2$ | $C_3$ | $C_4$ | $C_5$ | $C_6$ | $C_7$ |
|---|---|---|---|---|---|
| 4,6 | 7,4 | 14,6 | 36,8 | 31,0 | 5,5 |

### Beispiel 2

a) Erfindungsgemäßes Verfahren

Zu 3000 g 37%iger Formalinlösung und 246 g 86%iger Formoselösung gemäß Beispiel 1 werden unter Rühren bei 95° C 33,5 g PbO und danach in Abständen von jeweils 8 Minuten dreimal 5,7 g PbO zugesetzt. Nach 40 Minuten enthält die Lösung noch 4,7% HCHO, nach insgesamt 55 Minuten wird die Reaktion durch Kühlen bei 0% Formaldehydgehalt abgebrochen. Anfangs wird ein pH-Wert von 6,2, nach 3 Minuten ein solcher von 5,6 gemessen, zuletzt beträgt der pH 3,65. Die übliche Aufarbeitung ergibt mit 92% Ausbeute eine Formose mit folgenden Kenndaten: 71,6% Zucker (berechnet als Glucose) und 5,8% Wasser; Viskosität bei 20° C und 10% Wassergehalt: 28 600 mPas.

Komponentenverteilung nach Hydrierung (in %):

| $C_2$ | $C_3$ | $C_4$ | $C_5$ | $C_6$ | $C_7$ | $C_8$ |
|---|---|---|---|---|---|---|
| 1,4 | 4,0 | 8,8 | 33,8 | 39,7 | 10,9 | 1,5 |

b) Vergleichsversuch (gemäß US-Patent 2 224 910)

Werden nur 24,6 g der 86%igen Formoselösung als Co-Katalysator zusammen mit 3000 g der 37%igen Formaldehydlösung eingesetzt, müssen außer den ersten 33,5 g PbO im Abstand von je 7 Minuten noch neunmal je 5,6 g PbO (insgesamt 83,6 g PbO) zugesetzt werden, um nach 88 Minuten einen Restformaldehydgehalt von 4,6% im Reaktionsgemisch zu erhalten.

### Beispiel 3

In ein Gemisch aus 925 g einer 63,3%igen Formalinlösung (erhalten durch Einengen von 37%igem wäßrigem Formaldehyd) und 630 g der 88,2%igen Formose aus Beispiel 5 werden 27,9 g PbO (0,125 Mol) bei 95° C eingetragen. Der pH-Wert des Reaktionsgemisches steigt auf 5,8, die Innentemperatur nach 5 Minuten auf 104° C, nach 10 Minuten auf 107° C. Nach 20 Minuten Reaktionsdauer wird bei pH 4,4 und 0,3% Restformaldehydgehalt die Reaktion durch Kühlung abgebrochen. Nach der üblichen Aufarbeitung erhält man 1156 g Formose mit 66,0% Zucker (berechnet als Glukose) und 9,5% Wassergehalt.

Eine hydrierte Probe dieser Formose weist folgende Zusammensetzung (in%) auf:

| $C_2$ | $C_3$ | $C_4$ | $C_5$ | $C_6$ | $C_7$ | $C_8$ |
|---|---|---|---|---|---|---|
| 2,2 | 4,6 | 9,0 | 26,4 | 36,1 | 15,1 | 6,5 |

Beispiel 4

1000 g einer 18,5%igen Formalinlösung werden unter Rühren in einem Gefäß K1 auf 95°C erhitzt. Anschließend werden 500 g einer 74%igen Formoselösung gemäß Beispiel 1, danach 28,5 g Blei-II-acetattrihydrat (0,075 Mol) zugesetzt. Bei 101°C werden zum Reaktionsgemisch im Verlauf von ca. 20 Minuten 1,5 l heiße 34%ige Formalinlösung zugepumpt und zu Beginn des Pumpvorganges 19,0 g gelbes PbO auf einmal in die Formose-/Formaldehyd-Lösung eingetragen. Nach Ablauf der 20 Minuten wird das Zupumpen von Formalin in das Reaktionsgefäß K1 für 5 Minuten unterbrochen und in dieser Zeit aus K1 etwa 1,5 l Reaktionsgemisch in den Kolben K2 gesaugt. Darin wird unter Rühren und Rückfluß die Formosesynthese im Laufe von weiteren 20 Minuten zu Ende geführt.

Nach der Überführung des Formose-Formaldehyd-Gemisches von K1 nach K2 werden das Zupumpen von 1,5 l Formalin nach K1 sowie die darauf folgenden Operationen noch elfmal wiederholt.

Anfangs liegt der pH-Wert der Lösungen zwischen 4,3 und 5,0, später zwischen 4,8 und 5,4. Während der Reaktionszeit von 4 3/4 Stunden werden insgesamt 217 g Blei-II-oxid (0,97 Mol) zur Umsetzung von 20,45 kg 37%igem Formalin, entsprechend 7,57 kg wasserfreiem Formaldehyd, verbraucht. (Dies entspricht 28,7 g PbO oder 0,257 g-Äquivalenten PbO pro kg wasserfreiem HCHO). Der Restformaldehydgehalt in den einzelnen Fraktionen beträgt:

Fraktion-Nr./% HCHO:
1/3,6; 2/5,5; 3/3,6; 4/2,3; 5—13/0,6

Von dem Gemisch der Fraktionen 5 bis 13 wird ein Teil (A) über Ionenaustauscher vollentsalzt, der andere (B) durch 14stündige Elektrolyse entbleit (110 ppm Restblei). Die nach Einengen der wäßrigen Lösungen resultierenden Formosen enthalten (A) 69,8% Zucker bei 7,8% Wasser bzw. (B) 56,4% Zucker bei 2,1% Wasser. Komponentenverteilung der hydrierten Formosen laut Gaschromatographie (in %)

| | $C_2$ | $C_3$ | $C_4$ | $C_5$ | $C_6$ | $C_7$ | $C_8$ |
|---|---|---|---|---|---|---|---|
| A | 2,3 | 5,0 | 7,9 | 31,8 | 33,4 | 13,1 | 6,3 |
| B | 1,3 | 3,5 | 5,9 | 31,7 | 36,2 | 15,2 | 6,2 |

Beispiel 5

Zu 3000 g 37%igem Formalin und 220 g einer 100%igen Formose gemäß Beispiel 1 werden im Verlauf von 5 Minuten 58,2 g basisches Bleicarbonat (0,225 Grammatome Pb) bei 97°C zugesetzt. Der pH-Wert des Reaktionsgemisches steigt zunächst auf 4,4 und fällt während der weiteren Reaktionszeit auf 3,1 ab. Nach 150 Minuten wird die Umsetzung bei 3% Restformaldehydgehalt durch Kühlen mit Eiswasser und gleichzeitiges Zutropfen von 29 g Oxalsäuredihydrat in 250 g Wasser abgebrochen.

Nach Absaugen vom Niederschlag wird die Formose über Anionenaustauscher entsalzt und eingeengt. Man erhält 1233 g einer Formose mit 11,8% Wassergehalt und 60,7% Zucker (berechnet als Glucose).

Eine hydrierte Probe dieser Formose besitzt folgende Komponentenverteilung:

| | $C_2$ | $C_3$ | $C_4$ | $C_5$ | $C_6$ | $C_7$ |
|---|---|---|---|---|---|---|
| % | 6,1 | 7,4 | 12,6 | 30,4 | 36,6 | 6,8 |

Beispiel 6

a) Diskontinuierliches Verfahren

Durch Zugabe von 113,8 g Bleiacetattrihydrat (0,3 Mol) zu 2000 g 37%igem Formalin und 395 g 93,7%iger Formose aus Beispiel 1 bei 96°C wird die Formosesynthese bei pH 5,1 gestartet. Nach 25 Minuten ist bei 101°C der Formaldehydgehalt auf 19,5% und der pH-Wert auf 4,2 gesunken. Nach insgesamt 70 Minuten wird die Reaktion

bei pH 3,8 und 1,2% Restformaldehyd abgebrochen. Die übliche Aufarbeitung ergibt eine Formose mit 65,4% Zucker (als Glukose) bei 6,5% Wassergehalt. Die hydrierte Formose besteht aus folgenden Komponenten:

| $C_2$ | $C_3$ | $C_4$ | $C_5$ | $C_6$ | $C_7$ | $C_8$ |
|---|---|---|---|---|---|---|
| 3,8 | 5,5 | 9,8 | 25,3 | 40,5 | 12,3 | 3,0 |

b) Kontinuierliches Verfahren

296 g siedendes Wasser wird mit 204 g der 93,7%igen Formose aus Beispiel 1 und 100 g 37%igem Formalin versetzt. Bei 96°C werden 60 ml einer 33%igen Lösung von Bleiacetattrihydrat in Formalin zulaufen gelassen, wodurch sich der pH-Wert des Gemisches auf 4,8 einstellt. Nach 10 Minuten läßt man im Laufe von 20 Minuten gleichzeitig 650 g Formalin und 120 g

$$Pb(OOC-CH_3)_2 \cdot (3\,H_2O)$$

zutropfen. Der Formaldehydgehalt im Reaktionsgemisch steigt dabei auf 14,6%. Nun wird 30 Minuten lang die Formaldehydzufuhr unterbrochen und erst bei 3,5% HCHO und pH 4,2 wieder aufgenommen. Durch weiteren Bleiacetatzusatz wird der pH zwischen 4,2 und 4,3 gehalten, dabei wird der Formaldehyd so zudosiert, daß in der siedenden Lösung stets 2,0 bis 5,5% HCHO vorliegen.

Um 7 kg des 39%igen Formalins in 7 Stunden umzusetzen, werden insgesamt 900 ml der Bleiacetatlösung verbraucht. Jedesmal, wenn im Reaktionsgefäß ca. 3500 g Produkt vorliegen, werden Fraktionen von 1000 bis 1500 g entnommen.

Aus der Formoselösung werden die Bleiionen mit Oxalsäure gefällt. Entsalzen über Anionenaustauscher und Einengen ergibt 2355 g einer Formose mit 64% Zucker (berechnet als Glucose) und 8,1% Wasser.

**Patentansprüche**

1. Verfahren zur Herstellung von niedermolekularen Polyhydroxylverbindungen durch Selbstkondensation von Formaldehydhydrat in Gegenwart von löslichen oder schwer löslichen Verbindungen des zweiwertigen Bleis als Katalysator, deren gesättigte wäßrige Lösungen oder Suspensionen einen pH-Wert von über 4,5 aufweisen, dadurch gekennzeichnet, daß die Kondensationsreaktion in Gegenwart von

a) einer Menge an Katalysator, welche 0,1 bis 1 Grammäquivalenten Blei pro kg Formaldehyd entspricht,
b) mehr als 15 Gew.-% (bezogen auf Formaldehyd) an zur Endiolbildung befähigten Verbindungen als Co-Katalysator und gegebenenfalls
c) von niedermolekularen und/oder höhermolekularen Polyhydroxylverbindungen

durchgeführt wird, und daß in Abwesenheit weiterer organischer oder anorganischer Basen gearbeitet wird.

2. Verfahren nach Anspruch 1, dadurch gekennzeichnet, daß eine Menge an Katalysator, die 0,3 bis 0,6 Grammäquivalenten Blei pro kg Formaldehyd entspricht, und mehr als 40 Gew.-% (bezogen auf Formaldehyd) an Co-Katalysator eingesetzt werden.

3. Verfahren nach Anspruch 1 oder 2, dadurch gekennzeichnet, daß als Katalysator Bleioxid, Bleihydroxid, Bleicarbonat, basisches Bleicarbonat, Bleiacetat oder basisches Bleiacetat verwendet wird.

4. Verfahren nach Anspruch 1 bis 3, dadurch gekennzeichnet, daß als Co-Katalysator Formose eingesetzt wird.

5. Verfahren nach Anspruch 1 bis 4, dadurch gekennzeichnet, daß als Formaldehydquelle 25 bis 75gew.-%ige wäßrige oder alkoholische Formaldehydlösungen und/oder Paraformaldehyddispersionen eingesetzt werden.

6. Verfahren nach Anspruch 1 bis 4, dadurch gekennzeichnet, daß als Formaldehydquelle die bei der großtechnischen Formaldehydherstellung anfallenden formaldehydhaltigen Synthesegase dienen.

7. Verfahren zur Herstellung von gegebenenfalls zellförmigen Polyurethankunststoffen durch Umsetzung von

A) Polyisocyanaten mit
B) niedermolekularen Polyhydroxylverbindungen sowie gegebenenfalls
C) höhermolekularen Polyhydroxylverbindungen, weiteren Kettenverlängerungsmitteln, Treibmitteln, Katalysatoren und weiteren an sich bekannten Zusatzstoffen,

dadurch gekennzeichnet, daß als Komponente B die gemäß Anspruch 1 bis 6 erhaltenen Formosen und/oder deren Hydrierungsprodukte eingesetzt werden.

**Claims**

1. A process for the production of low molecular weight polyhydroxyl compounds comprising autocondensing formaldehyde hydrate in the presence of soluble or substantially insoluble compounds of divalent lead as catalyst, the

saturated aqueous solutions or suspensions of which have a pH value of more than 4.5, characterized in that the condensation reaction is carried out in the presence of

a) a quantity of catalyst which corresponds to from 0.1 to 1 gram equivalent of lead per kg of formaldehyde,
b) more than 15% by weight (based on formaldehyde) of compounds capable of enediol formation as co-catalyst, and optionally
c) low molecular weight and/or relatively high molecular weight polyhydroxyl compounds,

and in that the reaction is carried out in the absence of further organic or inorganic bases.

2. A process as claimed in Claim 1, characterized in that the catalyst is used in a quantity corresponding to from 0.3 and 0.6 gram equivalent of lead per kg of formaldehyde and the co-catalyst is used in a quantity of more than 40% by weight (based on formaldehyde).

3. A process according to Claim 1 or 2, characterized in that lead oxide, lead hydroxide, lead carbonate, basic lead carbonate, lead acetate or basic lead acetate is used as the catalyst.

4. A process according to Claim 1 to 3, characterized in that formose is used as the co-catalyst.

5. A process according to Claim 1 to 4, characterized in that from 25 to 75% by weight aqueous or alcoholic formaldehyde solutions and/or paraformaldehyde dispersions are used as the formaldehyde source.

6. A process according to Claim 1 to 4, characterized in that the formaldehyde-containing synthesis gases obtained in the production of formaldehyde on a commercial scale are used as the formaldehyde source.

7. A process for the production of optionally cellular polyurethane plastics by reacting

A) polyisocyanates with
B) low molecular weight polyhydroxyl compounds, and optionally
C) relatively high molecular weight polyhydroxyl compounds, further chain extenders, blowing agents, catalysts and other known additives,

characterized in that the formoses obtained according to Claim 1 to 6 and/or the hydrogenation products thereof are used as component B.

**Revendications**

1. Procédé de production de composés polyhydroxylés de bas poids moléculaire par autocondensation d'hydrate de formaldéhyde en présence d'un catalyseur formé de composés solubles ou peu solubles de plomb divalent, dont les solutions ou suspensions aqueuses saturées ont un pH supérieur à 4,5, caractérisé en ce que la réaction de condensation est conduite en présence:

(a) d'une quantité de catalyseur qui correspond à 0,1—1 équivalent-gramme de plomb par kilogramme de formaldéhyde,
(b) de plus de 15% en poids (par rapport au formaldéhyde) de composés aptes à former un endiol, comme cocatalyseur et, le cas échéant,
(c) de composés polyhydroxylés de bas poids moléculaire et/ou de poids moléculaire élevé

et en ce qu'on opère en l'absence d'autres bases organiques ou inorganiques.

2. Procédé suivant la revendication 1, caractérisé en ce qu'on utilise une quantité de catalyseur qui correspond à 0,3—0,6 équivalent-gramme de plomb par kilogramme de formaldéhyde et plus de 40% en poids (par rapport au formaldéhyde) de cocatalyseur.

3. Procédé suivant la revendication 1 ou 2, caractérisé en ce qu'on utilise comme catalyseur l'oxyde de plomb, l'hydroxyde de plomb, le carbonate de plomb, le carbonate de plomb basique, l'acétate de plomb ou l'acétate de plomb basique.

4. Procédé suivant l'une des revendications 1 à 3, caractérisé en ce qu'on utilise le formose comme cocatalyseur.

5. Procédé suivant l'une des revendications 1 à 4, caractérisé en ce qu'on utilise comme source de formaldéhyde des solutions aqueuses ou alcooliques à 25—75% en poids de formaldéhyde et/ou des dispersions de paraformaldéhyde.

6. Procédé suivant l'une des revendications 1 à 4, caractérisé en ce qu'on utilise comme source de formaldéhyde les gaz de synthèse contenant du formaldéhyde obtenus dans la production du formaldéhyde à l'échelle industrielle.

7. Procédé de production de matières plastiques du type polyuréthane éventuellement cellulaires, par réaction

(A) de polyisocyanates avec
(B) des composés polyhydroxylés de bas poids moléculaire ainsi que, le cas échéant,
(C) des composés polyhydroxylés de poids moléculaire élevé, d'autres agents d'allongement de chaîne, des agents porogènes, des catalyseurs et d'autres additifs connus,

caractérisé en ce qu'on utilise comme composant (B) les formoses obtenus conformément aux revendications 1 à 6 et/ou leurs produits d'hydrogénation.